# EUROPÄISCHE PATENTSCHRIFT

(11) **EP 0 356 949 B1**
(45) Veröffentlichungstag und Bekanntmachung des Hinweises auf die Patenterteilung: **06.04.1994**
(21) Anmeldenummer: 89115769.5
(22) Anmeldetag: 26.08.1989
(51) Int. Cl.: C07C 35/08, C07C 29/80

(54) **Verfahren zur Abtrennung von Cyclohexanol aus solches und aromatische Sulfonsäuren enthaltenden wässrigen Lösungen**
Process for the separation of cyclohexanol from aqueous solutions containing it as well as aromatic sulfonic acids
Procédé de séparation du cyclohexanol de solutions aqueuses le contenant et des acides sulfoniques aromatiques

(30) Priorität: 01.09.1988 DE 3829709
(43) Veröffentlichungstag der Anmeldung: 07.03.1990
(73) Patentinhaber: BASF Aktiengesellschaft, 67063 Ludwigshafen (DE)
(72) Erfinder: Gosch, Hans-Jürgen, Dr., D-6702 Bad Duerkheim (DE); Fischer, Rolf, Dr., D-6900 Heidelberg (DE); Luyken, Hermann, D-6700 Ludwigshafen (DE)

(56) Entgegenhaltungen:
- EP-A- 0 227 931
- GB-A- 2 151 224

## Beschreibung

Aus der europäischen Patentanmeldung 123 713 ist ein Verfahren bekannt, bei dem man Cyclohexanol durch Hydratisieren von Cyclohexen bei einer Temperatur von 50 bis 200°C in 5 bis 80 gew.-%igen Lösungen von aromatischen Sulfonsäuren in Wasser erhält. Um Korrosion zu verhindern, wird die Umsetzung z.B. in Gegenwart von Heteropolysäuren, Salzen oder Oxiden des Molybdäns, Wolframs oder Vanadins durchgeführt. Das durch Hydratisierung von Cyclohexen gebildete Cyclohexanol löst sich überwiegend in der aromatische Sulfonsäuren enthaltenden wäßrigen Phase und wird durch überschüssiges Cyclohexen nur zum geringen Teil aus dieser abgetrennt.

Entsprechend der japanischen Offenlegungsschrift 103.033/1987 werden für die Extraktion von Cyclohexanol aus aromatische Sulfonsäuren enthaltenden wäßrigen Lösungen aliphatische, cycloaliphatische oder aromatische Kohlenwasserstoffe empfohlen. Hierbei sind jedoch erhebliche Mengen an Extraktionsmittel erforderlich, um eine wirkungsvolle Extraktion zu erreichen. Dies ist technisch sehr aufwendig.

Nach einem anderen aus der japanischen Patentanmeldung 16.125/1968 bekannten Verfahren wird Cyclohexanol aus der Cyclohexanol und aromatische Sulfonsäuren enthaltenden wäßrigen Lösung zunächst durch Wasserdampfdestillation abgetrennt und aus dem erhaltenen wäßrigen Destillat Cyclohexanol mit Ether extrahiert und dann Cyclohexanol durch Verdampfen des Lösungsmittels gewonnen. Abgesehen davon, daß wiederum Lösungsmittel für die Extraktion mitverwendet werden müssen, wird bei der Wasserdampfdestillation ein nicht unerheblicher Teil Cyclohexanol wiederum in Cyclohexen zurückverwandelt. Deshalb wurde dort auch bereits versucht, die Cyclohexanol und aromatische Sulfonsäuren enthaltende wäßrige Ausgangslösung erheblich zu verdünnen, um die Rückspaltung zu Cyclohexen zu vermindern. Dies hat jedoch den Nachteil, daß die so erhaltene wäßrige aromatische Sulfonsäuren enthaltende Lösung ohne vorherige Aufkonzentrierung nicht mehr für die Hydratisierung verwendet werden kann.

Es war deshalb die technische Aufgabe gestellt, ein Verfahren zur Abtrennung von Cyclohexanol aus solches und aromatische Sulfonsäuren enthaltenden wäßrigen Lösungen zur Verfügung zu stellen, bei dem kein Extraktionsmittel erforderlich ist, die Rückspaltung von Cyclohexanol in Cyclohexen minimiert wird und eine wäßrige Lösung von aromatischen Sulfonsäuren, die unmittelbar für die Hydratisierung von Cyclohexen verwendbar ist, erhalten wird, und ferner keine behandlungsbedürftigen Abwässer anfallen.

Diese Aufgabe wird gelöst in einem Verfahren zur Abtrennung von Cyclohexanol aus solches und aromatische Sulfonsäuren enthaltenden wäßrigen Lösungen, die durch Hydratisieren von Cyclohexen in wäßrigen Lösungen von aromatischen Sulfonsäuren bei erhöhter Temperatur und anschließende Abtrennung von überschüssigem Cyclohexen erhalten wurden, durch Wasserdampfdestillation, wobei man
a) die Cyclohexanol und aromatische Sulfonsäuren enthaltende wäßrige Lösung im oberen Drittel einer ersten Kolonne und 1 bis 50 Gew.-Teile Wasserdampf je Gewichtsteil Cyclohexanol im unteren Drittel der Kolonne zuführt und am Kopf der Kolonne im wesentlichen aus Cyclohexanol, Cyclohexen und Wasser bestehende Brüden abzieht und als Sumpf eine wäßrige Lösung von aromatischen Sulfonsäuren erhält,
b) die im wesentlichen aus Cyclohexanol, Cyclohexen und Wasser bestehenden Brüden kondensiert und
   b1) eine cyclohexanolreiche Phase und
   b2) eine wäßrige Phase erhält
c) die wäßrige Phase b2) zur Erzeugung des in Stufe a benötigten Wasserdampfs verwendet und den gegebenenfalls verbleibenden Rest für die Hydratation von Cyclohexen verwendet und
d) aus der cyclohexanolreichen Phase b1) in einer zweiten Kolonne ein Gemisch aus Cyclohexen, Wasser und gegebenenfalls Cyclohexanol über Kopf abtrennt und Cyclohexanol als Sumpfprodukt gewinnt.

Das neue Verfahren hat den Vorteil, daß die Mitverwendung von Lösungsmitteln für die Extraktion vermieden wird. Weiter hat das neue Verfahren den Vorteil, daß die Rückspaltung von Cyclohexanol in Cyclohexen minimiert wird. Ferner hat das neue Verfahren den Vorteil, daß die anfallende wäßrige Lösung von aromatischen Sulfonsäuren ohne vorherige Aufkonzentrierung wiederum für die Hydratation von Cyclohexen verwendbar ist. Darüber hinaus hat das neue Verfahren den Vorteil, daß keine behandlungsbedürftigen Abwässer anfallen.

Erfindungsgemäß geht man von wäßrigen Lösungen aus, die Cyclohexanol und aromatische Sulfonsäuren gelöst enthalten. Solche Lösungen erhält man bei der Hydratisierung von Cyclohexen zu Cyclohexanol, wobei man Cyclohexen in wäßrigen Lösungen, die 5 bis 80 Gew.-% aromatische Sulfonsäuren, insbesondere Benzol- oder Naphthalinsulfonsäuren, die substituiert sein können, wie Benzolsulfonsäure, Toluolsulfonsäure oder Naphthalinsulfonsäure, ferner
Dodecylbenzolsulfonsäure, enthalten, bei einer Temperatur von z.B. 50 bis 200°C, insbesondere 70 bis 150°C, vorteilhaft unter einem Druck von 1 bis 10 bar, umsetzt. Vorteilhaft werden zusätzlich Molybdänsäure oder deren Salze, Vanadinoxid oder Vanadate in einer Menge z.B. von 0,001 bis 5 Gew.-%, bezogen auf die aromatischen Sulfonsäuren, mitverwendet. Darüber hinaus ist es auch empfehlenswert Heteropolysäuren wie Phosphormolybdänsäure, Phosphorwolframsäure, Phosphormolybdänwolframsäure oder Phosphormolybdänvanadinsäure mitzuverwenden. Geeignete Verfahren werden beispielsweise beschrieben in der europäischen Patentschrift 123 713 und der europäischen Patentanmeldung 206 631. Als Ausgangsstoffe sind neben Cyclohexen auch Cyclohexen enthaltende Kohlenwasserstoffgemische, z.B. Gemische aus Cyclohexen und Cyclohexan oder Cyclohexen, Cyclohexan und Benzol verwendbar. Aus dem bei der Hydratisierung erhaltenen Reaktionsgemisch scheidet sich als organische Phase überschüssiges Cyclohexen und gegebenenfalls Cyclohexan und Benzol ab, die geringe Mengen an Cyclohexanol und gegebenenfalls auftretende Nebenprodukte enthält. Nach Abtrennen der organischen Phase verbleibt eine wäßrige Phase, aus der Cyclohexanol abgetrennt werden muß. Eine typische Zusammensetzung der erhaltenen wäßrigen Phase ist beispielsweise 5 bis 10 Gew.-% Cyclohexanol, 40 bis 70 Gew.-% aromatische Sulfonsäuren und 20 bis 50 Gew. -% Wasser.

Erfindungsgemäß wird die Cyclohexanol und aromatische Sulfonsäuren enthaltende wäßrige Lösung in einer ersten Kolonne unter Mitverwendung von Wasserdampf destilliert. Hierbei wird die wäßrige Lösung von Cyclohexanol und aromatischen Sulfonsäuren dem oberen Drittel der ersten Kolonne und Wasserdampf dem unteren Drittel der Kolonne zugeführt. Je Gewichtsteil Cyclohexanol führt man 1 bis 50 Gew.-Teile Wasserdampf, insbesondere 4 bis 15 Gew.-Teile Wasserdampf zu. Geeignete Kolonnen sind beispielsweise Füllkörperkolonnen, Glockenbodenkolonnen, Tellerbödenkolonnen oder Siebbodenkolonnen. Die Kolonnen haben Zweckmäßig 1 bis 50, insbesondere 1 bis 5 theoretische Böden. Besonders bewährt hat es sich, wenn man für das Cyclohexanol eine kurze Verweilzeit z.B. von 1 bis 60 Minuten in der Kolonne einhält.

Die Abtrennung des Cyclohexanols erfolgt in der Regel bei einer Temperatur von 50 bis 170°C, insbesondere von 90 bis 110°C. Hierbei wendet man in der Regel Atmosphärendruck an. Es ist jedoch auch möglich, die Destillation unter vermindertem Druck, z.B. bis zu 20 mbar oder erhöhtem Druck, z.B. bis zu 10 bar durchzuführen.

Am Kopf der Kolonne werden im wesentlichen aus Cyclohexanol, Cyclohexen und Wasser bestehende Brüden abgezogen und im Sumpf der Kolonne erhält man eine wäßrige Lösung von aromatischen Sulfonsäuren.

In der Stufe b werden die am Kopf der Kolonne 1 erhaltenen, im wesentlichen aus Cyclohexanol, Cyclohexen und Wasser bestehenden Brüden kondensiert. Hierbei erhält man eine cyclohexanolreiche Phase (b1). Diese besteht im wesentlichen aus Cyclohexanol, Wasser und Cyclohexen. Eine typische Zusammensetzung ist beispielsweise 50 bis 90 Gew.-% Cyclohexanol, 0,1 bis 10 Gew.-% Cyclohexen und 12 bis 15 Gew.-% Wasser sowie geringe Mengen an Verunreinigungen. Zusätzlich erhält man eine wäßrige Phase (b2). Eine typische Zusammensetzung der Phase (b2) ist beispielsweise 93 bis 97 Gew.-% Wasser und 3 bis 7 Gew.-% Cyclohexanol.

Vorteilhaft werden die Brüden vor der Kondensation verdichtet, z.B. auf einen Druck von 1,5 bar und bei der Kondensation die Wärmeenergie in Stufe C, wie nachfolgend beschrieben, oder anderweitig verwendet.

In der Stufe c wird soviel der wäßrigen Phase (b2) verdampft, vorteilhaft unter Wärmeaustausch mit den verdichteten Brüden der Stufe b, die der benötigten Wasserdampfmenge für die erste Kolonne der Stufe a entspricht. Der verbleibende Rest an wäßriger Phase (b2) wird wiederum gegebenenfalls zusammen mit der Sumpfphase aus der Stufe a für die Hydratation von Cyclohexen verwendet.

Aus der cyclohexanolreichen Phase (b1) wird in Stufe d in einer zweiten Kolonne ein Gemisch aus Cyclohexen, Wasser und gegebenenfalls Cyclohexanol über Kopf abgetrennt und Cyclohexanol als Sumpfprodukt gewonnen. Geeignete Kolonnen sind beispielsweise Füllkörperkolonnen, Siebbodenkolonnen, Glockenbodenkolonnen oder Tellerbodenkolonnen. Solche Kolonnen haben vorteilhaft 5 bis 50, insbesondere 10 bis 25 theoretische Böden. Am Kopf der Kolonne hält man vorteilhaft eine Temperatur von 40 bis 150°C und im Sumpf eine Temperatur von 80 bis 200°C ein. In der Regel wird die Destillation unter Atmosphärendruck durchgeführt. Es ist jedoch auch möglich, verminderten Druck, z.B. bis zu 10 mbar oder erhöhten Druck, z.B. bis zu 5 bar, anzuwenden. Vorteilhaft wird die cyclohexanolreiche Phase (b1) dem mittleren Teil der Kolonne zugeführt. Die am Kopf der Kolonne erhaltenen Brüden werden kondensiert und scheiden sich in eine Wasser und gegebenenfalls Cyclohexanol enthaltende Cyclohexenphase (d1), die z.B. wiederum in die Hydratisierung zurückgeführt wird, und eine Cyclohexen und gegebenenfalls Cyclohexanol enthaltende wäßrige Phase (d2), die zweckmäßig wiederum mit der Phase (b2) vereinigt wird, und/oder direkt in die Hydratisierungsstufe zurückgeführt wird. Am Sumpf der Kolonne erhält man Cyclohexanol z.B. mit einem Gehalt von 99 bis 100 Gew.-%.

Besonders vorteilhaft entnimmt man im Seitenabzug z.B. im mittleren Drittel der zweiten Kolonne einen gasförmigen oder flüssigen Seitenstrom, der sich in zwei Phasen scheidet; in eine cyclohexanolreiche Phase (d3), mit einer typischen Zusammensetzung von 50 bis 90 Gew.-% Cyclohexanol, 0 bis 40 Gew.-% Cyclohexen und 1 bis 20 Gew.-% Wasser, die wiederum der zweiten Kolonne zugeführt wird. Die weiterhin anfallende wäßrige Phase (d4), die im wesentlichen aus Wasser und 0,1 bis 7 Gew.-% Cyclohexanol besteht, wird zweckmäßig mit der Phase (b2) des Kondensats aus der ersten Kolonne vereinigt, sofern die Wassermenge zur Dampferzeugung benötigt wird und/oder direkt gegebenenfalls zusammen mit dem Sumpf der ersten Kolonne, in die Hydratisierung zurückgeführt.

### Beispiel 1

### 1a) Abtrennung von Cyclohexanol durch Wasserdampf-Destillation

Auf den oberen Boden einer beheizbaren Glockenbodenkolonne mit 50 mm Bodendurchmesser und 3 Glockenböden wurden kontinuierlich 480 g/h einer Lösung aus 10 Gew.-Teilen Cyclohexanol, 53 Gew.-Teilen p-Toluolsulfonsäure, 2 Gew.-Teilen Molybdatophosphorsäure und 35 Gew.-Teilen Wasser zugeführt. Oberhalb des Kolonnensumpfes wurden gleichzeitig 580 g/h Wasserdampf eingeleitet. Das Gew.-Verhältnis von Cyclohexanol zu Wasserdampf betrug somit 1 : 12. Die Kolonne wurde so beheizt, daß die Innentemperatur 108°C betrug. Unter diesen Bedingungen wurden am Kopf der Kolonne 600 g/h Destillat erhalten. Das Destillat setzte sich aus 30 g/h einer cyclohexanolreichen und 570 g/h einer wäßrigen Phase zusammen. Die cyclohexanolreiche Phase bestand aus 83,6 Gw.-% Cyclohexanol, 3,3 Gew.-% Cyclohexen und 13,1 Gew.-% Wasser. Die wäßrige Phase enthielt 96 Gew.-% Wasser und 3,7 Gew.-% Cyclohexanol. Das Destillat enthielt somit 46 g/h Cyclohexanol und 0,9 g/h Cyclohexen. Am Sumpf der Kolonne liefen 460 g/h wäßrige Sulfonsäure-Lösung mit der Zusammensetzung: 56 Gew.-% p-Toluolsulfonsäure, 42 Gew.-% Wasser, 2 Gew.-% Molybdatophosphorsäure und 0,2 Gew.-% Cyclohexanol, ab. Damit wurden 96 Mol.-% des in der Sulfonsäure-Lösung enthaltenen Cyclohexanols abgetrennt; nur 2 Mol.-% wurden zu Cyclohexen gespalten.

### 1b) Reingewinnung von Cyclohexanol

Für die Reingewinnung des Cyclohexanols wurde eine beheizbare Glockenbodenkolonne mit 50 mm Bodendurchmesser und 30 Glockenböden verwendet. Auf dem von unten gerechnet 16. Boden der Kolonne wurden pro Stunde 114 g der in Beispiel 1a) nach Wasserdampfdestillation über Kopf gehenden cyclohexanolreichen Phase (83,6 % Cyclohexanol, 13,1 % Wasser, 3,3 % Cyclohexen) geleitet.

Am Kopf der Kolonne wurden nach Kondensation und Phasenscheidung pro Stunde folgende Produktmengen gewonnen:
3,7 g organische Phase (Rücklaufverhältnis 6.8) die zu 99,5 % Cyclohexen und zu 0,5 % aus Wasser bestand und in die Hydratisierungsstufe zurückgeführt werden kann.

3,0 g einer wäßrigen Phase, die zu 99,8 % aus Wasser und zu 0,2 % aus Cyclohexen bestand und in die Hydratisierungsstufe zurückgeführt werden kann.

Am von unten gerechnet 14. Boden der Kolonne wurde die gesamte Flüssigkeit abgezogen und in einem Phasenscheider getrennt. Hierbei wurden pro Stunde 132 g organische Phase erhalten, die zu 20,6 % aus Cyclohexen, zu 73,6 % aus Cyclohexanol und zu 5,8 % aus Wasser bestand. Die organische Phase wurde zurück auf den 13. Boden der Kolonne geleitet. Weiterhin wurden pro Stunde 12,3 g wäßrige Phase erhalten, die zu 96,8 % aus Wasser, zu 3,0 % aus Cyclohexanol und zu 0,1 % aus Cyclohexen bestand. Die wäßrige Phase kann in die Hydratisierungsstufe zurückgeführt werden.

Im Sumpfabzug der Kolonne wurden pro Stunde 95,2 g Cyclohexanol in einer Reinheit von 99,9 % erhalten.

### Beispiele 2 bis 4

Die Wasserdampfdestillation wurde, wie im Beispiel 1a beschrieben, durchgeführt. Die Ergebnisse in Tabelle 1 zeigen, daß mit abnehmender Wasserdampfmenge (bezogen auf Cyclohexanol) die abgetrennte Cyclohexanol-Menge sinkt und die Rückspaltungsrate des Cyclohexanols ansteigt (Ol = Cyclohexanol, En = Cyclohexen).

**Tabelle 1**

| Beispiel | Säure-Lösung (g/h) | H₂O-Dampf (g/h) | Gew.-Verhältnis Ol : H₂O-Dampf | Destillat g/h | | Sumpf g/h | | Ol-Spaltung (Mol .-%) |
|---|---|---|---|---|---|---|---|---|
| | | | | Ol | En | Ol | En | |
| 2 | 520 | 150 | 1 : 2,9 | 20 | 10 | 20 | 1 | 25 |
| 3 | 610 | 250 | 1 : 4,1 | 42 | 3 | 15 | 1 | 8 |
| 4 | 620 | 520 | 1 : 8,4 | 51 | 2 | 7 | 2 | 8 |
| ol = Cyclohexanol; En = Cyclohexen | | | | | | | | |

### Beispiele 5 bis 7

Die Destillation wurde, wie im Beispiel 1a beschrieben, durchgeführt, nur wurde eine beheizbare Glockenbodenkolonne mit 13 Glockenböden und einem Bodendurchmesser von 60 mm verwendet. Die Cyclohexanol/Sulfonsäure-Lösung wurde am 3. Boden von oben und der Dampf am 9. Boden von oben zugeführt. Die Temperatur in der Kolonne betrug 107°C. Für die Abtrennung des Cyclohexanols wurden verschiedene Verhältnisse von Cyclohexanol- zu Wasserdampfmenge verwendet. Die Ergebnisse sind in Tabelle 2 zusammengefaßt.

**Tabelle 2**

| Beispiel | Säure-Lösung (g/h) | H₂O-Dampf (g/h) | Gew.-Verhältnis Ol : H₂O-Dampf | Destillat g/h | | Sumpf g/h | | Ol-Spaltung (Mol .-%) |
|---|---|---|---|---|---|---|---|---|
| | | | | Ol | En | Ol | En | |
| 5 | 572 | 150 | 1 : 2,6 | 24 | 11 | 18 | 1 | 26 |
| 6 | 600 | 470 | 1 : 7,8 | 52 | 4 | 2 | 1 | 10 |
| 7 | 570 | 600 | 1 :10,5 | 53 | 3 | - | - | 6 |

### Beispiele 8 bis 11

Die Destillation wurde, wie in den Beispielen 5 bis 7 beschrieben, durchgeführt, nur daß der Dampf auf dem 13. Glockenboden, von oben gerechnet, eingeleitet wurde. Die Temperatur in der Kolonne betrug 108°C. Die Ergebnisse in Tabelle 3 zeigen im Vergleich mit denen in Tabelle 1, daß die Erhöhung der Zahl der praktischen Böden zu verstärkter Cyclohexen-Bildung und verringerter Cyclohexanol-Abtrennung führt.

**Tabelle 3**

| Beispiel | Säure-Lösung (g/h) | H₂O-Dampf (g/h) | Gew.-Verhältnis Ol : H₂O-Dampf | Destillat g/h | | Sumpf g/h | | Ol-Spaltung (Mol. -%) |
|---|---|---|---|---|---|---|---|---|
| | | | | Ol | En | Ol | En | |
| 8 | 530 | 135 | 1 : 2,5 | 17 | 14 | 17 | 2 | 36 |
| 9 | 560 | 220 | 1 : 3,9 | 29 | 14 | 9 | 1 | 32 |
| 10 | 570 | 450 | 1 : 7,9 | 50 | 6 | - | - | 13 |
| 11 | 600 | 600 | 1 :10 | 55 | 3 | - | 1 | 8 |

### Beispiel 12

Die Destillation wurde, wie in den Beispielen 9 bis 11 beschrieben, durchgeführt, nur betrug die Temperatur in der Kolonne 116°C. Das Ergebnis in Tabelle 4 zeigt im Vergleich zu Beispiel 9 (Tabelle 3), daß mit Erhöhung der Kolonnentemperatur die abgetrennte Cyclohexanol-Menge, aber auch die Cyclohexen-Menge steigt.

**Tabelle 4**

| Beispiel | Säure-Lösung (g/h) | H₂O-Dampf (g/h) | Gew.-Verhältnis Ol : H₂O-Dampf | Destillat g/h | | Sumpf g/h | | Ol-Spaltung (Mol. -%) |
|---|---|---|---|---|---|---|---|---|
| | | | | Ol | En | Ol | En | |
| 12 | 570 | 250 | 1 : 4,3 | 36 | 16 | - | 1 | 36 |

## Patentansprüche

1. Verfahren zur Abtrennung von Cyclohexanol aus solches und aromatische Sulfonsäuren enthaltenden wäßrigen Lösungen, die durch Hydratisieren von Cyclohexen in wäßrigen Lösungen von aromatischen Sulfonsäuren bei erhöhter Temperatur und anschließende Abtrennung von überschüssigem Cyclohexen erhalten wurden, durch Wasserdampfdestillation, dadurch gekennzeichnet, daß man
a) die Cyclohexanol und aromatische Sulfonsäuren enthaltende wäßrige Lösungen dem oberen Drittel einer ersten Kolonne und 1 bis 50 Gew.-Teile Wasserdampf je Gew.-Teil Cyclohexanol dem unteren Drittel der Kolonne zuführt und am Kopf der Kolonne im wesentlichen aus Cyclohexanol, Cyclohexen und Wasser bestehende Brüden abzieht und im Sumpf eine wäßrige Lösung von aromatischen Sulfonsäuren erhält
b) die im wesentlichen aus Cyclohexanol und Cyclohexen sowie Wasser bestehenden Brüden kondensiert und
b1) eine cyclohexanolreiche Phase und
b2) eine wäßrige Phase erhält
c) die wäßrige Phase (b2) zur Erzeugung des in Stufe a benötigten Wasserdampfs verwendet und den gegebenenfalls verbleibenden Rest für die Hydratation von Cyclohexen verwendet und
d) aus der cyclohexanolreichen Phase (b1) in einer zweiten Kolonne ein Gemisch aus Cyclohexen, Wasser und gegebenenfalls Cyclohexanol über Kopf abdestilliert und Cyclohexanol als Sumpfprodukt gewinnt.

2. Verfahren nach Anspruch 1, dadurch gekennzeichnet, daß man in der Stufe a eine Temperatur von 90 bis 110°C einhält.

3. Verfahren nach den Ansprüchen 1 und 2, dadurch gekennzeichnet, daß man in der Stufe a 4 bis 15 Gew.-Teile Wasserdampf je Gew.-Teil Cyclohexanol zuführt.

4. Verfahren nach den Ansprüchen 1 bis 3, dadurch gekennzeichnet, daß man die in der Stufe a anfallenden Brüden verdichtet und durch Wärmetausch mit der wäßrigen Phase (b2), die in der Stufe a erforderliche Menge an Wasserdampf erzeugt.

5. Verfahren nach den Ansprüchen 1 bis 4, dadurch gekennzeichnet, daß man in der Stufe d die cyclohexanolreiche Phase dem mittleren Drittel der zweiten Kolonne zuführt, am Kopf der Kolonne ein Gemisch aus Cyclohexen und Wasser erhält, im mittleren Drittel der Kolonne ein Gemisch aus Cyclohexanol und Wasser als Seitenabzug entnimmt und nach Phasentrennung die cyclohexanolreiche Phase in die Kolonne zurückführt und die wäßrige Phase mit der wäßrigen Phase (b1) vereinigt und Cyclohexanol als Sumpfprodukt gewinnt.

## Claims

1. A process for removing cyclohexanol from an aqueous solution containing it and an aromatic sulfonic acid, which has been obtained by hydrating cyclohexene in an aqueous solution of an aromatic sulfonic acid at an elevated temperature and subsequently removing excess cyclohexene, which comprises
a) feeding the aqueous solution which contains the cyclohexanol and the aromatic sulfonic acid into the upper third of a first column and from 1 to 50 parts by weight of steam per part by weight of cyclohexanol into the bottom third of the column, withdrawing at the top of the column a vapor which consists essentially of cyclohexanol, cyclohexene and water, and obtaining as the bottom product an aqueous solution of the aromatic sulfonic acid,
b) condensing the vapor which consists essentially of cyclohexanol, cyclohexene and water and
b1) obtaining a cyclohexanol-rich phase and
b2) an aqueous phase,
c) utilizing the aqueous phase (b2) for generating the steam required in stage a and any remainder for the hydration of cyclohexene, and
d) distilling off from the cyclohexanol-rich phase (b1) in a second column a mixture of cyclohexene and water with or without cyclohexanol as top product, obtaining cyclohexanol as bottom product.

2. A process as claimed in claim 1, wherein a temperature of from 90 to 110°C is maintained in stage a.

3. A process as claimed in claims 1 and 2, wherein from 4 to 15 parts by weight of steam per part by weight of cyclohexanol are fed in at stage a.

4. A process as claimed in claims 1 to 3, wherein the vapor obtained in stage a is compressed and the amount of steam required for stage a is produced by heat exchange with the aqueous phase (b2).

5. A process as claimed in claims 1 to 4, wherein in stage d the cyclohexanol-rich phase is fed into the middle third of the second column, a mixture of cyclohexene and water is obtained at the top of the column, a mixture of cyclohexanol and water is withdrawn from the side of the middle third of the column, and after phase separation the cyclohexanol-rich phase is recycled back into the column and the aqueous phase is combined with the aqueous phase (b1), and cyclohexanol is obtained as bottom product.

## Revendications

1. Procédé de séparation de cyclohexanol à partir de solutions aqueuses contenant celui-ci et des acides sulfoniques aromatiques, qui ont été obtenues par hydratation de cyclohexène dans des solutions aqueuses d'acides sulfoniques aromatiques à température élevée, puis par séparation du cyclohexène en excès, par entraînement à la vapeur, caractérisé en ce que
a) l'on alimente le tiers supérieur d'une première colonne de solutions aqueuses contenant du cyclohexanol et des acides sulfoniques aromatiques, et le tiers inférieur de la colonne de 1 à 50 parties en poids de vapeur d'eau par partie en poids de cyclohexanol, et l'on soutire en tête de la colonne des vapeurs essentiellement constituées de cyclohexanol, de cyclohexène et d'eau, et l'on obtient en fond de colonne une solution aqueuse d'acides sulfoniques aromatiques,
b) l'on condense les vapeurs essentiellement constituées de cyclohexanol et de cyclohexène ainsi que d'eau et
b1) l'on obtient une phase riche en cyclohexanol et
b2) une phase aqueuse,
c) l'on utilise la phase aqueuse b2) pour produire la vapeur d'eau nécessaire dans l'étape a) et l'on utilise la partie éventuellement restante pour l'hydratation du cyclohexène et
d) l'on chasse par distillation en tête d'une seconde colonne, à partir de la phase riche en cyclohexanol b1), un mélange de cyclohexène, d'eau et éventuellement de cyclohexanol, et l'on récupère du cyclohexanol comme produit de fond de colonne.

2. Procédé selon la revendication 1, caractérisé en ce que l'on fixe, dans l'étape a), une température de 90 à 110°C.

3. Procédé selon la revendication 1 ou 2, caractérisé en ce que l'on alimente, dans l'étape a), 4 à 15 parties en poids de vapeur d'eau par partie en poids de cyclohexanol.

4. Procédé selon l'une des revendications 1 à 3, caractérisé en ce que l'on condense les vapeurs résultant de l'étape a), et en ce que l'on produit la quantité de vapeur d'eau nécessaire dans l'étape a) par échange thermique avec la phase aqueuse b2).

5. Procédé selon l'une quelconque des revendications 1 à 4, caractérisé en ce que, dans l'étape d), on alimente le tiers médian de la seconde colonne de la phase riche en cyclohexanol, l'on obtient en tête de la colonne un mélange de cyclohexène et d'eau, l'on prélève dans le tiers médian de la colonne une fraction d'extraction latérale consistant en un mélange de cyclohexanol et d'eau, et l'on renvoie, après la séparation des phases, la phase riche en cyclohexanol dans la colonne, et l'on réunit la phase aqueuse et la phase aqueuse b1), et l'on récupère le cyclohexanol comme produit de fond de colonne.
